# EUROPEAN PATENT SPECIFICATION

(11) **EP 4 605 770 B1**
(45) Date of publication and mention of the grant of the patent: **10.12.2025**
(21) Application number: 24700914.5
(22) Date of filing: 12.01.2024
(51) Int. Cl.: A61B 34/20, A61B 17/00

(54) **SYSTEM FOR MAGNETIC TRACKING USING REUSABLE COMPONENTS**
SYSTEM ZUR MAGNETISCHEN VERFOLGUNG UNTER VERWENDUNG WIEDERVERWENDBARER KOMPONENTEN
SYSTÈME DE SUIVI MAGNÉTIQUE À L'AIDE DE COMPOSANTS RÉUTILISABLES

(43) Date of publication of application: 27.08.2025
(60) Divisional application: 25213409.3
(73) Proprietor: Brainlab SE, 81829 München (DE)
(72) Inventor: SCHAICH, Michael, 81829 Munich (DE); MLADENOVIC, Patra, 81829 Munich (DE); PIETERSMA, Elisah, 81829 Munich (DE); KOCH, Lukas, 81829 Munich (DE); BALDINI, Julia, 81829 Munich (DE)
(74) Representative: SSM Sandmair
(86) International application number: PCT/EP2024/050655
(87) International publication number: WO 2025/149167

(56) References cited:
- US-A1- 2012 035 468
- US-A1- 2019 246 944
- US-A1- 2020 237 446
- US-A1- 2022 047 295
- US-A1- 2022 378 516
- ORACO GALEAZZI: "La protesi totale di ginocchio con sistema Dash Brainlab - Dr. M. Ulivi", 4 June 2014 (2014-06-04), XP093133819, Retrieved from the Internet <URL:https://www.youtube.com/watch?v=xUL8wxyhD8k&rco=1> [retrieved on 20240221]

## Description

### FIELD OF THE INVENTION

The present invention relates to a system for magnetic tracking in a medical environment.

### TECHNICAL BACKGROUND

In the medical context, several approaches are known for tracking the position of a medical instrument. In this context, the term position means the location in up to three spatial dimensions and/or the orientation in up to three rotational dimensions.

One approach involves magnetic tracking in which sensors attached to the medical instrument detect a magnetic field generated by a field generator. The sensor output is typically transferred to a computing unit which calculates the position of the medical instrument, for example in a reference system of the field generator.

Some approaches use wireless transmission of the sensor data to the computing unit. However, medical instruments comprising wireless components are not suitable for steam sterilization since the wireless components might be damaged. On the other hand, the wireless components are too expensive for many disposable instruments. The present invention thus aims at re-using electronic components in magnetic tracking.

The present invention can for example be used for Cranial EM Navigation and ENT EM Navigation, both being products of Brainlab AG.

Aspects of the present invention, examples and exemplary steps and their embodiments are disclosed in the following. Different exemplary features of the invention can be combined in accordance with the invention wherever technically expedient and feasible.

Document US 2020/0237446 A1 discloses a surgical instrument having a position sensor that may be detachable, disposable, partially isolated from movement of the main body of the surgical instrument.

### EXEMPLARY SHORT DESCRIPTION OF THE INVENTION

The invention is defined in the claims. In the following, a short description of the specific features of the present invention is given which shall not be understood to limit the invention only to the features or a combination of the features described in this section.

The disclosed system comprises re-usable electronic components which are non-sterile, but encapsulated in a sterile, disposable hull. The hull can be a medical instrument or a separate entity which is mechanically connectable to a medical instrument.

### GENERAL DESCRIPTION OF THE INVENTION

In this section, a description of the general features of the present invention is given for example by referring to possible embodiments of the invention.

In general, the invention reaches the aforementioned object by providing a system for magnetic tracking in a medical environment, the system comprising a re-usable, non-sterile electronics unit. The electronics unit comprises a housing and at least one internal magnetometer sensor. A magnetometer sensor is a sensor that can detect the local strength of a magnetic field generated by a field generator at a sensor position. Any suitable kind of magnetometer sensor can be used. Each of the at least one internal magnetometer sensors is located at a predetermined position inside the electronics unit.

The electronics unit further comprises a sensor interface unit configured to process sensor data received from the at least one internal magnetometer sensor and/or an external magnetometer sensor external to the electronics unit. Processing for example involves digitizing an analog output from the magnetometer sensor. However, a magnetometer sensor could already provide a digital signal to the sensor interface unit. In this case, the sensor interface unit for example acts as micro controller that communicates with the magnetometer sensor and forwards the sensor data to the computing unit via a wireless communication module described below. Processing for example involves adding information identifying the magnetometer sensor which has measured the strength of the magnetic field in association with the measured strength. Alternatively, processing involves adding information on the position of the magnetometer sensor which has measured the strength of the magnetic field in association with the strength.

The electronics unit further comprises a wireless communication module configured to transmit the sensor data after processing by the sensor interface unit to a computing unit. The computing unit can be part of the system or not. The computing unit is configured to receive the sensor data from the electronics unit.

Still further, the electronics unit comprises a plurality of electric contacts reaching to the outside of the housing. Via the electric contacts, the electronics unit can be connected to other components. Reaching to the outside of the housing means that the electric contacts can be electrically contacted from the outside of the housing.

The system further comprises a sterile receiving element configured to receive the electronics unit in a hermetically sealed manner. Hermetically sealed does in this case mean that any contaminations the electronics unit might carry do not reach outside of the receiving element. The non-sterile electronics unit does thus not contaminate the sterile field and pose a risk to the patient. The sterile field is an area relative to the patient which is supposed to remain sterile during a treatment of the patient.

In this context, the term "sterile" means that the receiving element has undergone a sterilization process, for example gas sterilization with ethylene oxide gas, vapor sterilization or treatment with a sterilizing liquid. "Sterile" thus means a state which is generally accepted as being sterile in the medical field.

The advantage of such a system is that the electronics unit can be removed from the receiving element after use and be re-used after sterilization of the receiving element or with a new receiving element. In addition, the electronics unit can be used in combination with multiple kinds of receiving element. The receiving element can for example be a sterile case or a sterile medical instrument as explained below.

The receiving element can be disposable. In this context, the term "disposable" means that the receiving element is envisaged for one-time use only. The disposable receiving element is disposed after this single use. In this context, the system is cost effective since the electronics unit is not disposed together with the receiving element. This is particularly advantageous in that cheap medical instruments can be made to work with magnetic tracking.

The receiving element can also be a holder, such as a patient reference attached to a patient, for example to the head of the patient. The electronics unit attached to the patient via the receiving element can be used to track the position of the patient, or of a part of the patient. Depending on the use case, the holder need not necessarily be sterile, for example if it is located outside the sterile field.

The electronics unit is located inside the receiving element. Since the electronics unit is hermetically sealed inside the sterile receiving element, the system of the electronics unit and the receiving element is sterile.

The system preferably further comprises a battery. The battery stores electrical energy to power the sensor interface unit and the wireless communication module. The battery might be rechargeable.

In one implementation, the battery is located in the receiving element and the plurality of electric contacts comprise battery contacts for electrically connecting the battery to the electronics unit. The energy stored in the battery can thus be transported to the components within the electronics unit to drive them. In this implementation, the battery is not located in the electronics unit. The advantage of this implementation is that reusability of the electronics unit is not limited by the storage capacity of the battery since the electronics unit is powered externally.

In an alternative implementation, the battery is located inside the electronics unit. In this case, the electronics unit is completely self-contained and comprises all components necessary for use in magnetic tracking. In this alternative, the battery can be rechargeable from outside of the electronics unit, for example via the electric contacts.

As mentioned above, there are many possible types of receiving element.

In one embodiment, the receiving element is a sterile case comprising a mechanical connector for connecting the sterile case to a medical instrument. The mechanical connector is capable of rigidly attaching the sterile case to the medical instrument. In this document, "rigidly attaching" means that a first component rigidly attached to a second component has a fixed relative position to the second component
In this embodiment, the electronics unit is rigidly attached or held inside of the sterile case. This means that, overall, the electronics unit is rigidly attached to the medical instrument via the sterile case and the mechanical connector. The relative position between the medical instrument and the electronics unit is thus constant during use.

An advantage of the present embodiment is that the non-sterile electronics unit inside the sterile casing can be attached to a sterile medical instrument. In this case, the combination of the medical instrument and the electronics unit inside the sterile casing is sterile.

As an option, the system further comprises a medical instrument having a mechanical connector connectable to the mechanical connector of the sterile case. In use, the sterile case is rigidly attached to the medical instrument via the mechanical connectors of the sterile case and of the medical instrument. The system thus comprises the medical instrument, the sterile casing and the electronics unit.

The electronics unit can be rigidly held in the sterile case via the electric contacts of the electronics unit. In addition or as an alternative, the sterile case might have a cavity having a mating shape to the shape of the electronics unit, such that the electronics unit is held in the sterile case in a rigid manner.

In this embodiment, the relative position between the electronics unit and the medical instrument is constant during use. This means that the medical instrument can be tracked by tracking the electronics unit. In this case, tracking involves that the electronics unit sends the output data of the internal magnetometer sensor(s) inside the electronics unit to the computing unit. The computing unit determines the position of the electronics unit, and thus of the medical instrument, in a tracking reference system, like a reference system of the field generator, from the predetermined positions of the at least one magnetometer sensor in the electronics unit. The technology of magnetic tracking is known and thus not described here in detail.

In some use cases, it is desired to track a particular point of interest of the medical instrument. In this case, the electronics unit has to be calibrated with the point of interest of the medical instrument. This means that the location of the point of interest relative to the electronics unit is determined such that the computing unit can calculate the location of the point of interest of the medical instrument in the tracking reference system.

Calibration of the electronics unit with the point of interest of the medical instrument can be performed in many ways. In one approach, the point of interest is brought into a defined location in the tracking reference system, like a predetermined point on the surface of the field generator. The location of the point of interest of the medical instrument in the tracking reference system is thus known. The position of the electronics unit is measured using the magnetic tracking system. The computing unit calculates the calibration from those two information.

In another approach, a calibration tool is used. The calibration tool comprises a reference point against which the point of interest of the medical instrument is held. The position of the calibration tool in the tracking reference system is measured, for example using the magnetic tracking system. The geometry of the calibration tool is known to the computing unit. In this case, the geometry means the location of the reference point on the calibration tool. The computing unit calculates the calibration from the measured positions of the electronics unit and of the calibration tool in the tracking reference system and from the known geometry of the calibration tool.

The calibration tool can for example be a device having a portion with a shape inverse to the shape of the medical instrument around the point of interest or a notch for receiving the point of interest. The medical instrument can thus be mated with the calibration tool for calibration. As another example, the calibration tool can be a tracked pointer. The tip of the pointer is held against the point of interest of the medical instrument for calibration.

In one embodiment, the receiving element is a medical instrument. The subsequent embodiments are particularly useful if the receiving element is a medical instrument, but they are not limited to this. A medical instrument can for example be a pointer, a stylet or a suction device.

In this embodiment, the electronics unit is not located in a sterile casing which in turn is attached to a medical instrument, but directly in the medical instrument. The electronics unit is rigidly attached to the medical instrument in analogy to the embodiment described above in which the receiving element is a sterile case.

The medical instrument being the receiving unit is sterile and accommodates the electronics unit inside such that contaminations of the electronics unit do not reach outside of the medical instrument.

The medical instrument may have electric contacts to mate with the electric contacts of the electronics unit such that the electronics unit can be connected to electronic components of the medical instrument.

In one embodiment, the sensor interface unit is configured to process user input data, for example in terms of a trigger signal input to the sensor interface unit by operation of a button of the medical instrument. The button of the medical instrument, or any other user input acquisition unit, is connected to the sensor interface unit via the electric contacts of the medical instrument and the electric contacts of the electronics unit. The sensor interface unit provides the information on the user input, for example the information that the button of the medical instrument was pushed, to the wireless communication module, which in turn transmits this information to the computing unit. The computing unit can then trigger a function, like sampling of a point, making a screenshot or any other function associated with the user input.

In one embodiment, the system further comprises at least one external magnetometer sensor in the receiving element, wherein the plurality of electric contacts of the electronics unit comprise data contacts connectable to the at least one magnetometer sensor in the receiving element and connected to the sensor interface unit in the electronics unit.

The magnetometer sensor(s) in the receiving element are referred to as external magnetometer sensor(s) since they are external to the electronics unit. The external magnetometer sensors may be located at different positions of the receiving element. This means that the distance between the external magnetometer sensors can be larger than the distance between internal magnetometer sensors in the electronics unit, which means that it is possible to detect the strength of the magnetic field at positions distributed over a larger area, which can improve the tracking result.

Therefore, in one embodiment, the sensor interface unit is configured to process the sensor data received from a magnetometer sensor in the receiving element. The electronics unit thus processes the output of the external magnetometer sensor and transmits it to the computing unit. This can be in addition to the output of one or more internal magnetometer sensors within the electronics unit. The computing unit can thus receive and process the output of one or more external magnetometer sensors and optionally the output of one or more internal magnetometer sensors.

In one embodiment, the system further comprises a calibration data memory in the receiving element, the calibration data memory storing calibration data of the receiving element. In one implementation, the calibration data represents the location of a point of interest of the receiving element relative to the at least one magnetometer sensor of the receiving element. Alternatively, the calibration data represents the positions of the at least one magnetometer sensor relative to the location of the point of interest of the receiving element. With the calibration data memory, it is not necessary to calibrate the electronics unit with the medical instrument since the information required to calculate the location of the point of interest of the medical instrument in the tracking reference system from the positions of the external magnetometer sensors(s) in the tracking reference system is comprised in the calibration data.

The calibration data memory, like any other memory that is mentioned in this document, can be of any suitable kind, such as a RAM, a ROM, a flash memory or any kind of chip or storage that can be read.

**In** one implementation, the receiving element comprises calibration data contacts connected to the calibration data memory and configured to mate with electric contacts of the electronics unit. The electronics unit can thus read the calibration data from the calibration data memory and transmit them to the computing unit via the wireless communication module. The computing unit can then use the calibration data to calculate the location of the point of interest of the receiving element.

The calibration data can represent the location of the point of interest of the receiving element relative to the cavity which accommodates the electronics unit or relative to the electric contacts of the receiving element which contact the electric contacts of the electronics unit. The combination of such calibration data and the predetermined position of an internal magnetometer sensor in the electronics unit defines the location of the point of interest of the receiving element relative to the position of the internal magnetometer sensor. The computing unit might use this information for calculating the location of the point of interest of the receiving element if the sensor data of the internal magnetometer sensor is transmitted to the computing unit.

In one embodiment, the calibration data represents a unique identifier of the receiving element. The electronics unit transmits the unique identifier to the computing unit. The computing unit may retrieve stored calibration data, for example from a database, corresponding to the unique identifier. The stored calibration data can represent the location of a point of interest of the receiving element relative to the at least one magnetometer sensor of the receiving element, the location of the point of interest of the receiving element relative to the cavity which accommodates the electronics unit or relative to electric contacts of the receiving element which contact the electric contacts of the electronics unit, a combination thereof or further data.

Receiving the unique identifier at the computing unit may indicate that a medical instrument associated with this unique identifier is actually used. The computing unit may store the unique identifier together with a counter representing the number of times the unique identifier has been received, and may thus store how often the medical instrument has been used. The computing unit may output a warning signal, like an optical or acoustical signal, if the maximum number of allowable uses of the medical instrument has been reached or exceeded. One example are single use instruments which may only be used once.

The computing unit may increase the stored counter not every time the unique identifier is received since the unique identifier might be received multiple times during the same procedure. The counter may for example only be increased if a minimum time has elapsed since the previous increase of the counter when receiving the unique identifier.

In one embodiment, the electronics unit further comprises at least one inertia sensor. An inertia sensor can detect accelerations acting on the electronics unit, which may include gravity, in particular the direction of gravity. The sensor interface unit can process the output of the inertia sensor and transmit the processed result to the computing unit via the wireless communication unit. The computing unit can use the data delivered by the inertia sensor, in addition to the data delivered by the magnetometer sensor(s), to calculate the position of the electronics unit or of the receiving element in the tracking reference system.

In addition or as an alternative, at least one inertia sensor is provided in the receiving element. Those inertia sensors are referred to as external inertia sensors since there are not located inside the electronics unit. In this case, the electronic contacts of the electronics unit comprise inertia sensor contacts connectable to the at least external inertia sensor, either directly or via one or more conductors. The data collected by the external inertia sensor(s)can be processed in the same manner as the data collected by the inertia sensor(s) inside the electronics unit.

According to the present invention, the sensor interface unit is configured to determine in which kind of receiving element the electronics unit is located. In particular, the sensor interface unit is configured to determine whether the receiving element is a sterile case, a medical instrument or a holder.

If the receiving unit is a sterile case or a holder, no external magnetometer sensor(s) and typically no calibration data memory are connected to the electric contacts of the electronics unit. The sensor interface unit recognizes that there is no input of such sensors and no calibration data can be read, and thus determines that the receiving element is a sterile case or a holder. If the sensor interface unit detects sensor data input to the electric contacts or a calibration data memory connected to the electronics unit, it determines that the receiving element is a medical instrument.

On the other hand, each kind of receiving unit could comprise a memory which stores the kind of receiving element, for example in terms of the unique identifier. The sensor interface unit reads the data from the memory and determines the kind of receiving element.

The sensor interface unit may determine the kind of receiving element using mechanical properties. For example, the receiving element may comprise a mechanical pin which presses against a predetermined position on the surface of the electronics unit and the electronics unit detects that pressure is excerted thereon. The electronics unit may thus comprise one or more pressure detectors, each of them configured to detect pressure on a predetermined area of the electronics unit.

One pressure detector may be sufficient if it is only used to distinguish between a sterile case and a holder, while a medical instrument is identified via data stored in a memory of the medical instrument. For example, the holder may comprise a pin to excert pressure on the electronics unit and the sterile case may not. However, the electronics unit may comprise multiple pressure sensors and the receiving element may comprise a combination of pins that identifies the kind of receiving element.

As an alternative, the receiving element may comprise an interruption element. The interruption element is configured to interrupt a circuit in the electronics unit, such as an electric circuit or a light circuit which guides light. The kind of receiving element can be identified from whether the circuit is interrupted or not. In analogy to the previous example, the receiving element can comprise one or more interruption elements and the electronics unit may comprise on or more circuits which can be interrupted.

The sensor interface unit may transmit the determined kind of receiving element, or the unique identifier if applicable, to the computing unit via the wireless communication module. The computing unit can then trigger an appropriate application, function or workflow.

If the electronics unit is located in a sterile case, the computing unit can for example trigger a calibration workflow in which the location of a point of interest of a medical instrument relative to the electronics unit is measured as described above.

If the electronics unit is located in a holder, the computing unit can use the electronics unit as a reference for the patient. This means that a reference system associated with the electronics unit in the holder is used for other spatial information, such as the position of a point of interest of a medical instrument. It is possible to calculate a transformation from the reference system of the field generator into the reference system of the electronics unit in the holder.

In one embodiment, the electronics unit comprises a light emitting element. The sensor interface unit may provide information to a user by operation of the light emitting element, for example based on information received from the computing unit via the wireless communication module or based on internal information within the sensor interface unit. In this embodiment, the receiving element comprises a transparent or translucent part such that the light emitted by the light emitting element can be recognized from outside of the receiving element.

### DEFINITIONS

In this section, definitions for specific terminology used in this disclosure are offered which also form part of the present disclosure.

### Pointer

A pointer is a rod which comprises one or more - advantageously, two - magnetometer sensors fastened to it and which can be used to measure off individual co-ordinates, for example spatial co-ordinates (i.e. three-dimensional co-ordinates), on a part of the body, wherein a user guides the pointer (for example, a part of the pointer which has a defined and advantageously fixed position with respect to the at least one magnetometer sensors attached to the pointer) to the position corresponding to the co-ordinates, such that the position of the pointer can be determined by using a surgical navigation system to detect the positions of the magnetometer sensor on the pointer. The relative location between the magnetometer sensors of the pointer and the part of the pointer used to measure off co-ordinates (for example, the tip of the pointer) is for example known. The surgical navigation system then enables the location (of the three-dimensional co-ordinates) to be assigned to a predetermined body structure, wherein the assignment can be made automatically or by user intervention.

### Fixed (relative) position

A fixed position, which is also referred to as fixed relative position, in this document means that two objects which are in a fixed position have a relative position which does not change unless this change is explicitly and intentionally initiated. A fixed position is in particular given if a force or torque above a predetermined threshold has to be applied in order to change the position. This threshold might be 10 N or 10 Nm. In particular, the position of a sensor device remains fixed relative to a target while the target is registered or two targets are moved relative to each other. A fixed position can for example be achieved by rigidly attaching one object to another. The spatial location, which is a part of the position, can in particular be described just by a distance (between two objects) or just by the direction of a vector (which links two objects). The alignment, which is another part of the position, can in particular be described by just the relative angle of orientation (between the two objects).

### BRIEF DESCRIPTION OF THE DRAWINGS

In the following, the invention is described with reference to the appended figures which give background explanations and represent specific embodiments of the invention. The scope of the invention is however not limited to the specific features disclosed in the context of the figures, wherein
- Fig. 1: shows an electronics unit;
- Fig. 2: shows the electronics unit of figure 1 in a sterile case attached to a medical instrument;
- Fig. 3: shows another medical instrument in which the electronics unit of figure 1 is incorporated;
- Fig. 4: shows a tracking environment using the medical instrument of figure 3; and
- Fig. 5: shows a holder for the electronics unit of figure 1.

### DESCRIPTION OF EMBODIMENTS

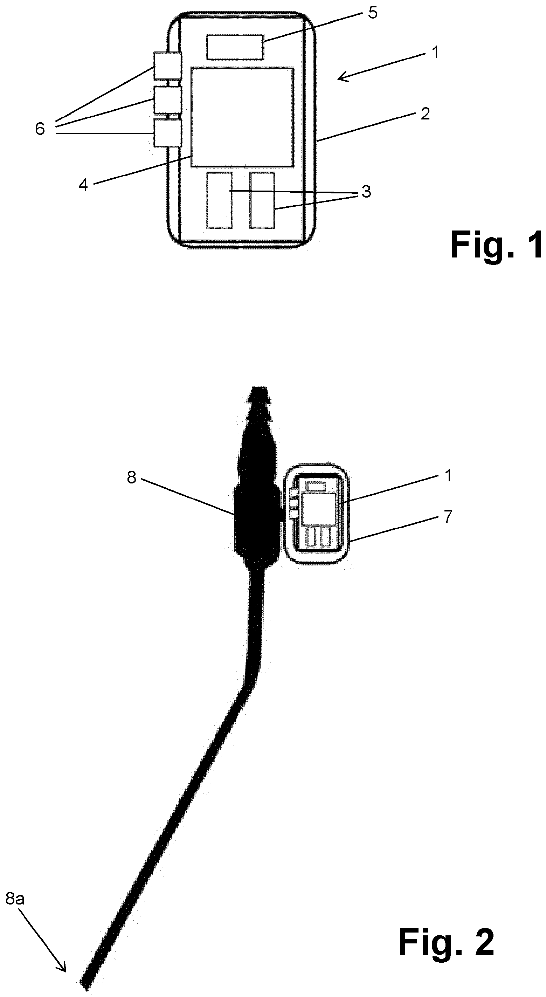

Figure 1 shows a re-usable, non-sterile electronics unit 1. The electronics unit 1 comprises a housing 2 in which two magnetometer sensors 3, a sensor interface unit 4 and a wireless communication module 5 are disposed. Instead of two magnetometer sensors 3, only one magnetometer sensor or more than two magnetometer sensors can be provided. As an option, the electronics unit 1 can comprise one or more inertia sensors (not shown).

The electronics unit 1 further comprises electric contacts 6 reaching to the outside of the housing 2 such that they can be connected to components external to the housing 2. In the present example, three electric contacts 6 are shown, but there can be more or fewer electric contacts as required. External components can include one or more of a battery to power the electronics unit 1, a magnetometer sensor and a memory.

The magnetometer sensors 3 are electrically connected to the sensor interface unit 4 such that the sensor interface unit 4 can receive data or signals generated by the magnetometer sensors 3. The magnetometer sensors 3 in particular output data or a signal corresponding to the strength of a magnetic field sensed by the magnetometer sensors 3. The magnetometer sensors 3 are provided at predetermined positions in the housing 2.

The sensor interface unit 4 receives the output of a magnetometer sensor, such as the magnetometer sensors 3, and processes it. **If** the magnetometer sensor outputs an analog signal, the sensor interface unit 4 performs analog-digital conversion of the analog signal and samples the converted signal. **If** the magnetometer sensor outputs a digital signal, the sensor interface unit receives and processes this digital signal. The sensor interface unit 4 transmits the processed data via the wireless communication module 5.

The electronics unit 1 can comprise a battery within the housing 2 to power the sensor interface unit 4 and the wireless communication module 5. It optionally powers the magnetometer sensors 3 if they require power to detect the strength of the magnetic field. In addition or as an alternative, the electronics unit 1 is connected to an external battery, which is external to the electronics unit 1, via the electric contacts 6 to supply power to the electronics unit 1.

Figure 2 shows a system comprising the electronics unit 1 and a sterile casing 7 as a receiving element for the electronics unit 1. The sterile casing 7 encapsulates the electronics unit 1 such that it is hermetically sealed therein. This means that contaminations of the electronics unit 1 do not reach outside of the casing 7.

The casing 7 has a cavity with a shape which exactly matches the shape of the housing 2 of the electronics unit 1 such that the electronics unit 1 has a fixed position relative to the casing 7. The medical instrument 8 and the casing 7 each have a mechanical connector (not shown) which form a mechanical interface and can mate with each other to rigidly attach the casing 7 to the medical instrument 8. The two mechanical connectors can form a (releasable) geometry, such as a positive locking, or just form contact surfaces which are connected via means like a screw or a bolt.

In the example shown in figure 2, a battery to power the electronics unit 2 can be located within the electronics unit 2 or within the casing 7. In the latter case, the battery is connected to the electronics unit 2 via the electric contacts 6.

With the electronics unit 1 firmly held by the casing 7 and the casing 7 being rigidly attached to the medical instrument 8, the position of the electronics unit 1, and in particular of the magnetometer sensors 3 in the electronics unit 1, relative to the medical instrument 8 is constant, such that the position of the medical instrument 8 is associated with the position of the electronics unit 1. The position of the medical instrument 8 can thus be derived from the position of the electronics unit 1, which in turn can be derived from the locations of the magnetometer sensors 3 of the electronics unit 1 and their positions therein.

The medical instrument 8 has a point of interest in terms of its tip 8a. In some use cases, the location of the tip 8a might be of interest. In this case, the location of the tip relative to the electronics unit 1 is measured, for example by measuring the position of the electronics units 1 using the output of its magnetometer sensors 3 and measuring the location of the tip 8a using a calibration device such as a pointer. This is also referred to as calibration. By tracking the electronics unit 1 and applying the calibration, the location of the tip 8a can be tracked.

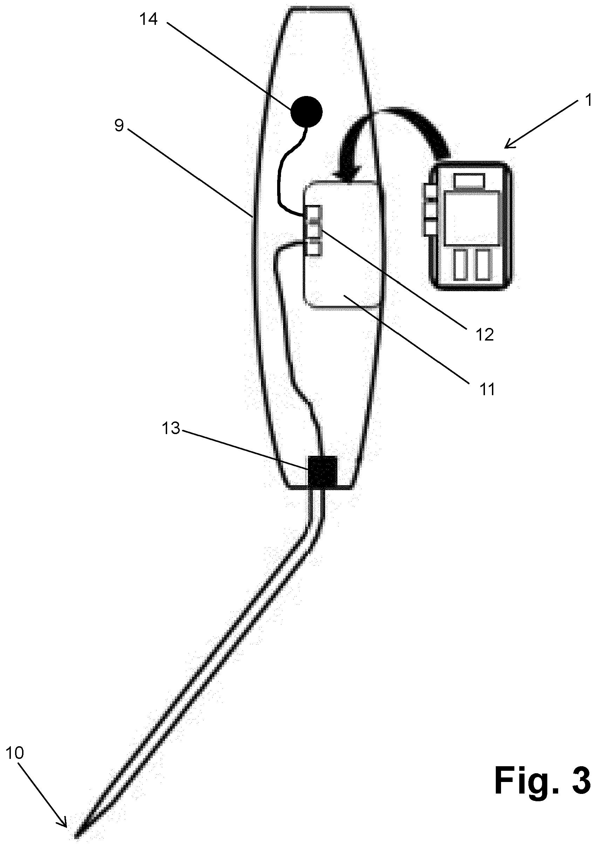

Figure 3 shows another instrument 9 having a sealable cavity 11 which accommodates the electronics unit 1 therein in a hermetically sealed manner as indicated by the arrow. The electric contacts 6 of the electronics unit are connected to electric contacts 12 of the medical instrument 9.

The medical instrument 9 comprises a magnetometer sensor 13 connected to the electronics unit 1 via the electric contacts 12 of the medical instrument 9 and the electric contacts 6 of the electronics unit 1. The sensor interface unit 4 reads and processes the output of the magnetometer sensor 13 and transmits the result via the wireless communication module 5. The sensor interface unit 4 associates each data item representing the strength of the magnetic field as detected by a magnetometer sensor 13 with the identification of the magnetometer sensor 13 which has measured the corresponding strength or with a position of said magnetometer sensor 13 relative to the electronics unit 1. The tracking system described later can thus identify at which location of the medical instrument 9 which strength of the magnetic field was measured.

The medical instrument 9 further comprises a calibration data memory 14 connected to the electronics unit 1 via the electric contacts 12 of the medical instrument and the electric contacts 6 of the electronics unit 1. The calibration data memory stores calibration data which is read by the sensor interface unit 4 and transmitted via the wireless communication module 5. The calibration data for example represents the position of the magnetometer sensor 13 in the medical instrument 9, in particular relative to a point of interest of the medical instrument 9, like the tip 10 of the medical instrument 9. Alternatively, the calibration data for example represents the location of the tip 10 relative to the position of the magnetometer sensor 13.

The medical instrument 9 may comprise two or more magnetometer sensors 13 and the calibration data memory 14 preferably stores calibration data for each of the magnetometer sensors 13 of the medical instrument 9.

When placed in the cavity 11, the position of the electronics unit 1 relative to the medical instrument is optionally fixed, for example by the electric contacts 6 of the electronics unit 1 and the electric contacts 12 of the medical instrument 9 engaging with each other, by partial or full form fit of the electronics unit 1 in the cavity 11 or a combination thereof. The position of the electronics unit 1 relative to the medical instrument 9 is known, for example from the geometry of the medical instrument 9 or a calibration of the system. The sensor interface unit 4 then optionally also reads and processes the output of the magnetometer sensors 3 and transmits the result via the wireless communication module 5 in analogy to the output of the magnetometer sensors 13. This in particular means that the identification of the magnetometer sensor 3 which has measured the strength of the magnetic field or the position of the magnetometer sensor 3 is transmitted. The tracking system has thus additional information to track the medical instrument 9.

With the calibration data memory 14, the medical instrument 9 is pre-calibrated and there is no need to calibrate the medical instrument 9 in situ. In addition, the magnetometer sensors 13 can be distributed over the medical instrument 9 such that the strength of the magnetic field can be measured at locations which are spatially distinct.

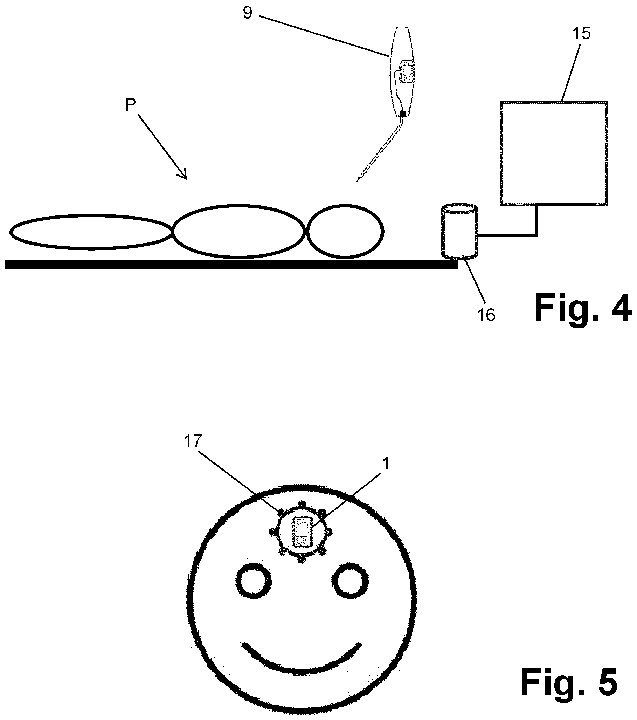

Figure 4 shows a tracking environment in which a tracking system comprising a computing unit 15 and a field generator 16 is employed. The field generator generates a variable magnetic field to be detected by the magnetometer sensors. The computing unit 15 receives the data transmitted by the wireless communication module 5 and calculates the location of the magnetometer sensors in a tracking reference system, which is for example defined relative to the field generator 16. A receiver for receiving the data transmitted by the wireless communication module 5 of the electronics unit 1 may be provided in the field generator 16 or in the computing unit 15.

In the example shown in figure 4, the medical instrument 9 is used to sample points of a patient P. The computing unit 15 calculates the locations of the sampled points in the tracking reference system.

Figure 5 shows a holder 17 for an electronics unit 1. The holder 17 is attached to the patient P, for example to the forehead of the patient P. The holder 17 is for example attached using an adhesive or a tape. With the electronics unit 1 attached to the patient P, movement of the patient relative to the field generator 16 can be tracked. In addition, the patient P can be registered in the tracking reference system, for example by sampling multiple points on the surface of the patient using the medical instrument 9. If the patient P is registered in the tracking reference system, the tracking system can calculate the position of a tracked object, like the medical instrument 9, relative to the patient P.

The electronics unit 1 can optionally comprise one or more inertia sensors which detect accelerations of the electronics unit 1. The output of the inertia sensor(s) is processed by the sensor interface unit 4 and transmitted to the computing unit 15 via the wireless communication module 5. The computing unit 15 uses the output of the inertia sensor(s) when calculating the position of the electronics unit 1. Instead of or in addition to the inertia sensor(s) in the electronics unit, one or more inertia sensors can be provided in the sterile casing 7, in the medical instrument 9 or in the holder 17. Those inertia sensors are connected to the electronics unit 1 via the electric contacts 6.

## Claims

1. A system for magnetic tracking in a medical environment, the system comprising a re-usable, non-sterile electronics unit (1) comprising
- a housing (2),
- at least one internal magnetometer sensor (3),
- a sensor interface unit (4) configured to process sensor data received from the at least one internal magnetometer sensor (3) and/or an external magnetometer sensor (13) external to the electronics unit (1),
- a wireless communication module (5) configured to transmit the sensor data after processing by the sensor interface unit (4) to a computing unit (15), and
- a plurality of electric contacts (6) reaching to the outside of the housing (2),
wherein the system further comprises a sterile receiving element (7, 9) configured to receive the electronics unit (1) in a hermetically sealed manner and
wherein the electronics unit (1) is located inside the receiving element (7, 9), **characterized in that**
the sensor interface unit (4) is configured to determine in which kind of receiving element (7, 9) the electronics unit (1) is located.

2. The system of claim 1, further comprising a battery.

3. The system of claim 2, wherein the battery is located in the receiving element (7, 9) and the plurality of electric contacts (6) comprise battery contacts for electrically connecting the battery to the electronics unit (1).

4. The system of any one of claims 1 to 3, wherein the sensor interface unit (4) is configured to process the sensor data received from a magnetometer sensor (3) in the electronics unit (1).

5. The system of any one of claims 1 to 4, wherein the receiving element is a sterile case (7) comprising a mechanical connector for connecting the sterile case (7) to a medical instrument (8).

6. The system of claim 5, further comprising a medical instrument (8) having a mechanical connector connectable to the mechanical connector of the sterile case (7).

7. The system of any one of claims 1 to 4, further comprising at least one external magnetometer sensor (13) in the receiving element (7, 9) and external to the electronics unit (1), wherein the plurality of electric contacts (6) of the electronics unit (1) comprise data contacts connectable to the at least one magnetometer sensor (13) in the receiving element (7, 9) and connected to the sensor interface unit (4) in the electronics unit (1).

8. The system of claim 7, wherein the sensor interface unit (4) is configured to process the sensor data received from a magnetometer sensor (13) in the receiving element (7, 9).

9. The system of any one of claims 5 to 8, further comprising a calibration data memory (14) in the receiving element (9), the calibration data memory (14) storing calibration data of the receiving element (9).

10. The system of claim 9, wherein the calibration data represents the location of a point of interest (10) of the receiving element (9) relative to the at least one magnetometer sensor (13) of the receiving element (9).

11. The system of claim 9, wherein the calibration data represents a unique identifier of the receiving element (9).

12. The system of any one of claims 7 to 11, wherein the receiving element is a medical instrument (9).

13. The system of any one of claims 1 to 12, wherein the electronics unit (1) further comprises at least one inertia sensor.

14. A method of preparing a sterile receiving element (7, 9) for magnetic tracking in a medical environment, comprising the step of disposing a re-usable, non-sterile electronics unit (1) as defined in claim 1 in the sterile receiving element (7, 9) in a hermetically sealed manner.

## Patentansprüche

1. Ein System zum magnetischen Tracking in einer medizinischen Umgebung, wobei das System eine wiederverwendbare, nicht sterile Elektronikeinheit (1) umfasst, die Folgendes umfasst
- ein Gehäuse (2),
- mindestens einen internen Magnetometersensor (3),
- eine Sensorschnittstelleneinheit (4), die so konfiguriert ist, dass sie Sensordaten verarbeitet, die von dem mindestens einen internen Magnetometersensor (3) und/oder einem externen Magnetometersensor (13) außerhalb der Elektronikeinheit (1) empfangen werden,
- ein drahtloses Kommunikationsmodul (5), das so konfiguriert ist, dass es die Sensordaten nach der Verarbeitung durch die Sensorschnittstelleneinheit (4) an eine Recheneinheit (15) überträgt, und
- eine Vielzahl von elektrischen Kontakten (6), die bis zur Außenseite des Gehäuses (2) reichen,
wobei das System ferner ein steriles Aufnahmeelement (7, 9) umfasst, das dazu eingerichtet ist, die Elektronikeinheit (1) hermetisch abgedichtet aufzunehmen, und
wobei sich die Elektronikeinheit (1) innerhalb des Aufnahmeelements (7, 9) befindet, **dadurch gekennzeichnet, dass**
die Sensorschnittstelleneinheit (4) dazu ausgelegt ist, zu bestimmen, in welcher Art von Aufnahmeelement (7, 9) sich die Elektronikeinheit (1) befindet.

2. Das System nach Anspruch 1, das ferner eine Batterie umfasst.

3. Das System nach Anspruch 2, wobei sich die Batterie in dem Aufnahmeelement (7, 9) befindet und die mehreren elektrischen Kontakte (6) Batteriekontakte zum elektrischen Verbinden der Batterie mit der Elektronikeinheit (1) umfassen.

4. Das System nach einem der Ansprüche 1 bis 3, wobei die Sensorschnittstelleneinheit (4) so konfiguriert ist, dass sie die von einem Magnetometersensor (3) in der Elektronikeinheit (1) empfangenen Sensordaten verarbeitet.

5. Das System nach einem der Ansprüche 1 bis 4, wobei das Aufnahmeelement ein steriles Gehäuse (7) ist, das einen mechanischen Verbinder zum Verbinden des sterilen Gehäuses (7) mit einem medizinischen Instrument (8) umfasst.

6. Das System nach Anspruch 5, das ferner ein medizinisches Instrument (8) mit einem mechanischen Verbinder umfasst, der mit dem mechanischen Verbinder des sterilen Gehäuses (7) verbindbar ist.

7. Das System nach einem der Ansprüche 1 bis 4, das ferner mindestens einen externen Magnetometersensor (13) im Aufnahmeelement (7, 9) und außerhalb der Elektronikeinheit (1) umfasst, wobei die mehreren elektrischen Kontakte (6) der Elektronikeinheit (1) Datenkontakte umfassen, die mit dem mindestens einen Magnetometersensor (13) im Aufnahmeelement (7, 9) verbunden werden können und mit der Sensorschnittstelleneinheit (4) in der Elektronikeinheit (1) verbunden ist.

8. Das System nach Anspruch 7, wobei die Sensorschnittstelleneinheit (4) so konfiguriert ist, dass sie die von einem Magnetometersensor (13) im Aufnahmeelement (7, 9) empfangenen Sensordaten verarbeitet.

9. Das System nach einem der Ansprüche 5 bis 8, das ferner einen Kalibrierungsdatenspeicher (14) im Aufnahmeelement (9) umfasst, wobei der Kalibrierungsdatenspeicher (14) Kalibrierungsdaten des Aufnahmeelements (9) speichert.

10. Das System nach Anspruch 9, wobei die Kalibrierungsdaten die Position eines Punktes von Interesse (10) des Aufnahmeelements (9) relativ zu dem mindestens einen Magnetometersensor (13) des Aufnahmeelements (9) darstellen.

11. Das System nach Anspruch 9, wobei die Kalibrierungsdaten eine eindeutige Kennung des Aufnahmeelements (9) darstellen.

12. Das System nach einem der Ansprüche 7 bis 11, wobei das Aufnahmeelement ein medizinisches Instrument (9) ist.

13. Das System nach einem der Ansprüche 1 bis 12, wobei die Elektronikeinheit (1) ferner mindestens einen Trägheitssensor umfasst.

14. Ein Verfahren zur Vorbereitung eines sterilen Aufnahmeelements (7, 9) für das magnetische Tracking in einer medizinischen Umgebung, umfassend den Schritt des Anbringens einer wiederverwendbaren, nicht sterilen Elektronikeinheit (1) gemäß Anspruch 1 in dem sterilen Aufnahmeelement (7, 9) in einer hermetisch versiegelten Weise.

## Revendications

1. Système de suivi magnétique dans un environnement médical, le système comprenant une unité électronique (1) réutilisable et non stérile comprenant
- un boîtier (2),
- au moins un capteur magnétomètre (3) interne,
- une unité interface de capteur (4) configurée pour traiter des données de capteur reçues à partir de l'au moins un capteur magnétomètre (3) interne et/ou d'un capteur magnétomètre (13) externe à l'unité électronique (1),
- un module de communication sans fil (5) configuré pour transmettre les données de capteur après traitement par l'unité interface de capteur (4) à une unité informatique (15), et
- une pluralité de contacts électriques (6) s'étendant jusqu'à l'extérieur du boîtier (2),
dans lequel le système comprend en outre un élément de réception (7, 9) stérile configuré pour recevoir l'unité électronique (1) de manière hermétiquement scellée et
dans lequel l'unité électronique (1) est située à l'intérieur de l'élément de réception (7, 9), **caractérisé en ce que**
l'unité interface de capteur (4) est configurée pour déterminer le type d'élément de réception (7, 9) dans lequel l'unité électronique (1) est située.

2. Système selon la revendication 1, comprenant en outre une batterie.

3. Système selon la revendication 2, dans lequel la batterie est située dans l'élément de réception (7, 9) et la pluralité de contacts électriques (6) comprend des contacts de batterie pour raccorder électriquement la batterie à l'unité électronique (1).

4. Système selon l'une quelconque des revendications 1 à 3, dans lequel l'unité interface de capteur (4) est configurée pour traiter les données de capteur reçues à partir d'un capteur magnétomètre (3) dans l'unité électronique (1).

5. Système selon l'une quelconque des revendications 1 à 4, dans lequel l'élément de réception est un étui stérile (7) comprenant un raccord mécanique pour raccorder l'étui stérile (7) à un instrument médical (8).

6. Système selon la revendication 5, comprenant en outre un instrument médical (8) présentant un raccord mécanique pouvant être raccordé au raccord mécanique de l'étui stérile (7).

7. Système selon l'une quelconque des revendications 1 à 4, comprenant en outre au moins un capteur magnétomètre (13) externe dans l'élément de réception (7, 9) et externe à l'unité électronique (1), dans lequel la pluralité de contacts électriques (6) de l'unité électronique (1) comprend des contacts de données pouvant être raccordés à l'au moins un capteur magnétomètre (13) dans l'élément de réception (7, 9) et raccordés à l'unité interface de capteur (4) dans l'unité électronique (1).

8. Système selon la revendication 7, dans lequel l'unité interface de capteur (4) est configurée pour traiter les données de capteur reçues à partir d'un capteur magnétomètre (13) dans l'élément de réception (7, 9).

9. Système selon l'une quelconque des revendications 5 à 8, comprenant en outre une mémoire de données d'étalonnage (14) dans l'élément de réception (9), la mémoire de données d'étalonnage (14) stockant des données d'étalonnage de l'élément de réception (9).

10. Système selon la revendication 9, dans lequel les données d'étalonnage représentent l'emplacement d'un point d'intérêt (10) de l'élément de réception (9) par rapport à l'au moins un capteur magnétomètre (13) de l'élément de réception (9).

11. Système selon la revendication 9, dans lequel les données d'étalonnage représentent un identifiant unique de l'élément de réception (9).

12. Système selon l'une quelconque des revendications 7 à 11, dans lequel l'élément de réception est un instrument médical (9).

13. Système selon l'une quelconque des revendications 1 à 12, dans lequel l'unité électronique (1) comprend en outre au moins un capteur d'inertie.

14. Procédé de préparation d'un élément de réception (7, 9) stérile pour le suivi magnétique dans un environnement médical, comprenant l'étape consistant à disposer une unité électronique (1) réutilisable et non stérile telle que définie dans la revendication 1 dans l'élément de réception (7, 9) stérile de manière hermétiquement scellée.
